(19) Europäisches Patentamt
European Patent Office
Office européen des brevets

(11) EP 1 664 013 B1

(12) EUROPEAN PATENT SPECIFICATION

(45) Date of publication and mention of the grant of the patent:
**14.05.2008 Bulletin 2008/20**

(21) Application number: **04784821.3**

(22) Date of filing: **23.09.2004**

(51) Int Cl.:
*C07D 321/00* (2006.01) *A61K 31/365* (2006.01)
*C07D 313/00* (2006.01) *C07D 409/12* (2006.01)
*A61P 19/08* (2006.01) *A61P 19/10* (2006.01)
*A61P 35/00* (2006.01) *A61P 3/14* (2006.01)
*A61P 19/02* (2006.01)

(86) International application number:
**PCT/US2004/031120**

(87) International publication number:
**WO 2005/030749 (07.04.2005 Gazette 2005/14)**

(54) **CALCILYTIC COMPOUNDS**

KALZILYTISCHE VERBINDUNGEN

COMPOSES CALCILYTIQUES

(84) Designated Contracting States:
**AT BE BG CH CY CZ DE DK EE ES FI FR GB GR HU IE IT LI LU MC NL PL PT RO SE SI SK TR**
Designated Extension States:
**HR LT LV**

(30) Priority: **24.09.2003 US 506001 P**

(43) Date of publication of application:
**07.06.2006 Bulletin 2006/23**

(73) Proprietor: **GLAXO GROUP LIMITED**
**Greenford,**
**Middlesex UB6 0NN (GB)**

(72) Inventor: **MARQUIS, Robert, W., Jr.**
**Collegeville, PA 19426 (US)**

(74) Representative: **Connell, Anthony Christopher et al**
**GlaxoSmithKline**
**Corporate Intellectual Property (CN9.25.19)**
**980 Great West Road**
**Brentford, Middlesex TW8 9GS (GB)**

(56) References cited:
**US-B1- 6 353 019**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 664 013 B1

## Description

### FIELD OF INVENTION

The present invention relates to novel calcilytic compounds, pharmaceutical compositions containing these compounds and their use as calcium receptor antagonists.

In mammals, extracellular $Ca^{2+}$ is under rigid homeostatic control and regulates various processes such as blood clotting, nerve and muscle excitability, and proper bone formation. Extracellular $Ca^{2+}$ inhibits the secretion of parathyroid hormone ("PTH") from parathyroid cells, inhibits bone resorption by osteoclasts, and stimulates secretion of calcitonin from C-cells. Calcium receptor proteins enable certain specialized cells to respond to changes in extracellular $Ca^{2+}$ concentration.

PTH is the principal endocrine factor regulating $Ca^{2+}$ homeostasis in the blood and extracellular fluids. PTH, by acting on bone and kidney cells, increases the level of $Ca^{2+}$ in the blood. This increase in extracellular $Ca^{2+}$ then acts as a negative feedback signal, depressing PTH secretion. The reciprocal relationship between extracellular $Ca^{2+}$ and PTH secretion forms an important mechanism maintaining bodily $Ca^{2+}$ homeostasis.

Extracellular $Ca^{2+}$ acts directly on parathyroid cells to regulate PTH secretion. The existence of a parathyroid cell surface protein which detects changes in extracellular $Ca^{2+}$ has been confirmed. See Brown et al., Nature 366:574, 1993. In parathyroid cells, this protein, the calcium receptor, acts as a receptor for extracellular $Ca^{2+}$, detects changes in the ion concentration of extracellular $Ca^{2+}$, and initiates a functional cellular response, PTH secretion.

Extracellular $Ca^{2+}$ influences various cell functions, reviewed in Nemeth et al., Cell Calcium 11:319, 1990. For example, extracellular $Ca^{2+}$ plays a role in parafollicular (C-cells) and parathyroid cells. See Nemeth, Cell Calcium 11: 323, 1990. The role of extracellular $Ca^{2+}$ on bone osteoclasts has also been studied. See Zaidi, Bioscience Reports 10: 493, 1990.

Various compounds are known to mimic the effects of extra-cellular $Ca^{2+}$ on a calcium receptor molecule. Calcilytics are compounds able to inhibit calcium receptor activity, thereby causing a decrease in one or more calcium receptor activities evoked by extracellular $Ca^{2+}$. Calcilytics are useful as lead molecules in the discovery, development, design, modification and/or construction of useful calcium modulators, which are active at $Ca^{2+}$ receptors. Such calcilytics are useful in the treatment of various disease states characterized by abnormal levels of one or more components, e.g., polypeptides such as hormones, enzymes or growth factors, the expression and/or secretion of which is regulated or affected by activity at one or more $Ca^{2+}$ receptors. Target diseases or disorders for calcilytic compounds include diseases involving abnormal bone and mineral homeostasis.

Abnormal calcium homeostasis is characterized by one or more of the following activities: an abnormal increase or decrease in serum calcium; an abnormal increase or decrease in urinary excretion of calcium; an abnormal increase or decrease in bone calcium levels (for example, as assessed by bone mineral density measurements); an abnormal absorption of dietary calcium; an abnormal increase or decrease in the production and/or release of messengers which affect serum calcium levels such as PTH and calcitonin; and an abnormal change in the response elicited by messengers which affect serum calcium levels.

Thus, calcium receptor antagonists offer a unique approach towards the pharmacotherapy of diseases associated with abnormal bone or mineral homeostasis, such as hypoparathyroidism, osteosarcoma, periodontal disease, fracture healing, osteoarthritis, rheumatoid arthritis, Paget's disease, humoral hypercalcemia associated with malignancy and fracture healing, and osteoporosis.

### SUMMARY OF THE INVENTION

The present invention comprises novel calcium receptor antagonists represented by Formula (I) hereinbelow and their use as calcium receptor antagonists in the treatment of a variety of diseases associated with abnormal bone or mineral homeostasis, including but not limited to hypoparathyroidism, osteosarcoma, periodontal disease, fracture healing, osteoarthritis, rheumatoid arthritis, Paget's disease, humoral hypercalcemia associated with malignancy and fracture healing, and osteoporosis.

The present invention further provides the use of an effective amount of a compound of Formula (I), indicated herein below, in the manufacture of a medicament for antagonizing calcium receptors in an animal, including humans in need thereof.

The present invention further provides the use of an effective amount of a compound of Formula (I), indicated herein below, in the manufacture of a medicament for increasing serum parathyroid levels in an animal, including humans in need thereof.

## DETAILED DESCRIPTION OF THE INVENTION

The compounds of the present invention are selected from Formula (I) herein below:

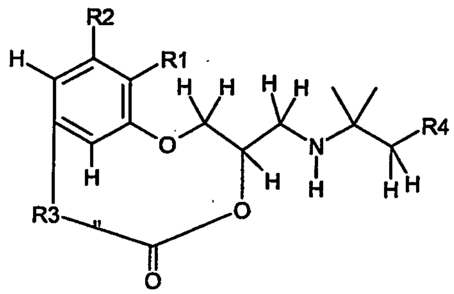

R1 is CN, or halogen
R2 is halogen or H
R3 is $C_{3-7}$ alkyl, or $C_{3-7}$ alkenyl; optionally substituted
R4 is selected from the group consisting of aryl, fused aryl, dihydro, tetrahydro fused aryl, and heteroaryl, unsubstituted or substituted, with any substituent selected from the group consisting of OH, halogen, $C_{1-4}$ alkyl, $C_{1-4}$ alkoxy, $CF_3$, $OCF_3$, CN and $NO_2$.

As used herein, "alkyl" refers to an optionally substituted hydrocarbon group joined by single carbon-carbon bonds and having 1-20 carbon atoms joined together. The alkyl hydrocarbon group may be linear or branched. Preferably, substituents on optionally substituted alkyl are selected from the group consisting of aryl, $CO_2R$, $CO_2NHR$, OH, OR, CO, $NH_2$, halo, $CF_3$, $OCF_3$ and $NO_2$, wherein R represents H, $C_{1-4}$ alkyl, $C_{3-6}$ cycloalkyl, $C_{2-5}$ alkenyl, $C_{2-5}$ alkynyl, heterocycloalkyl, or aryl. Additional substituents are selected from F, Cl, Br, I, N, S and O. Preferably, no more than three substituents are present. More preferably, the alkyl has 1-12 carbon atoms and is unsubstituted. Preferably, the alkyl group is linear.

As used herein "cycloalkyl" refers to optionally substituted 3-7 membered carbocyclic rings wherein any substituents are selected from the group consisting of, F, Cl, Br, I, $N(R_4)_2$, $SR_4$ and $OR_4$, unless otherwise indicated.

As used herein, "aryl" refers to an optionally substituted aromatic group with at least one ring having a conjugated pi-electron system, containing up to two conjugated or fused ring systems. Aryl includes carbocyclic aryl, and biaryl groups, all of which may be optionally substituted. Preferred aryl include phenyl and naphthyl. More preferred aryl include phenyl. Preferred substituents are selected from the group consisting of halogen, $C_{1-4}$ alkyl, $OCF_3$, $CF_3$, OMe, CN, $OSO_2$ R and $NO_2$, wherein R represents $C_{1-4}$ alkyl or $C_{3-6}$ cycloalkyl. As used herein, "heteroaryl" refers to an aryl ring containing 1,2 or 3 heteroatoms such as N, S, or O.

As used herein, "alkenyl" refers to an optionally substituted hydrocarbon group containing at least one carbon-carbon double bond and containing up to 5 carbon atoms joined together. The alkenyl hydrocarbon chain may be straight or branched. Any substituents are selected from the group consisting of halogen, $C_{1-4}$ alkyl, $OFF_3$, $CF_3$, OMe, CN, $OSO_2$ R and $NO_2$, wherein R represents $C_{1-4}$ alkyl or $C_{3-6}$ cycloalkyl.

As used herein, "alkynyl" refers to an optionally substituted hydrocarbon group containing at least one carbon-carbon triple bond between the carbon atoms and containing up to 5 carbon atoms joined together. The alkynyl hydrocarbon group may be straight-chained, branched or cyclic. Any substituents are selected from the group consisting of halogen, $C_{1-4}$ alkyl, $OCF_3$, $CF_3$, OMe, CN, $OSO_2$ R and $NO_2$, wherein R represents $C_{1-4}$ alkyl or $C_{3-6}$ cycloalkyl.

The compounds of the present invention may contain one or more asymmetric carbon atoms and may exist in racemic and optically active forms. All of these compounds and diastereomers are contemplated to be within the scope of the present invention.

Preferred compounds of the present inventions include:

(R)-4-[(2-Indan-2-yl-1,1-dimethyl-ethylamino)-methyl]-6-oxo-2,5-dioxa-bicyclo[9.3.1]pentadeca-1(14),11(15),12-triene-14-carbonitrile;
(R)-13,14-Difluoro-4-[(2-indan-2-yl-1,1-dimethyl-ethylamino)-methyl]-2,5-dioxa-bicyclo[9.3.1]pentadeca-1(14),11(15),12-trien-6-one;
(R)-4-{[2-(5-Chloro-thiophen-2-yl)-1,1-dimethyl-ethylamino]-methyl}-13,14-difluoro-2,5-dioxa-bicyclo[9.3.1]pentadeca-1(14),11(15),12-trien-6-one;
(R)-4-{[2-(5-Chloro-thiophen-2-yl)-1,1-dimethyl-ethylamino]-methyl}-6-oxo-2,5-dioxa-bicyclo[9.3.1]pentadeca-1(14),11(15),12-triene-14-carbonitrile;

(R)-14-Bromo-4-[(2-indan-2-yl-1,1-dimethyl-ethylamino)-methyl]-2,5-dioxa-bicyclo[9.3.1]pentadeca-1(14),11(15),12-trien-6-one; and

(R)-14-Bromo-4-{[2-(5-chloro-thiophen-2-yl)-1,1-dimethyl-ethylamino]-methyl}-2,5-dioxa-bicyclo[9.3.1]pentadeca-1(14),11(15),12-trien-6-one.

Pharmaceutically acceptable salts are non-toxic salts in the amounts and concentrations at which they are administered.

Pharmaceutically acceptable salts include acid addition salts such as those containing sulfate, hydrochloride, fumarate, maleate, phosphate, sulfamate, acetate, citrate, lactate, tartrate, methanesulfonate, ethanesulfonate, benzenesulfonate, p-toluenesulfonate, cyclohexylsulfamate and quinate. A preferred salt is a hydrochloride. Pharmaceutically acceptable salts can be obtained from acids such as hydrochloric acid, maleic acid, sulfuric acid, phosphoric acid, sulfamic acid, acetic acid, citric acid, lactic acid, tartaric acid, malonic acid, methanesulfonic acid, ethanesulfonic acid, benzenesulfonic acid, p-toluenesulfonic acid, cyclohexylsulfamic acid, fumaric acid, and quinic acid.

Pharmaceutically acceptable salts also include basic addition salts such as those containing benzathine, chloroprocaine, choline, diethanolamine, ethylenediamine, meglumine, procaine, aluminum, calcium, lithium, magnesium, potassium, sodium, ammonium, alkylamine, and zinc, when acidic functional groups, such as carboxylic acid or phenol are present.

The present invention provides compounds of Formula (I) above, which can be prepared using standard techniques. An overall strategy for preparing preferred compounds described herein can be carried out as described in this section. The examples, which follow, illustrate the synthesis of specific compounds. Using the protocols described herein as a model, one of ordinary skill in the art can readily produce other compounds of the present invention.

All reagents and solvents were obtained from commercial vendors. Starting materials were synthesized using standard techniques and procedures.

**Synthetic Scheme 1.**

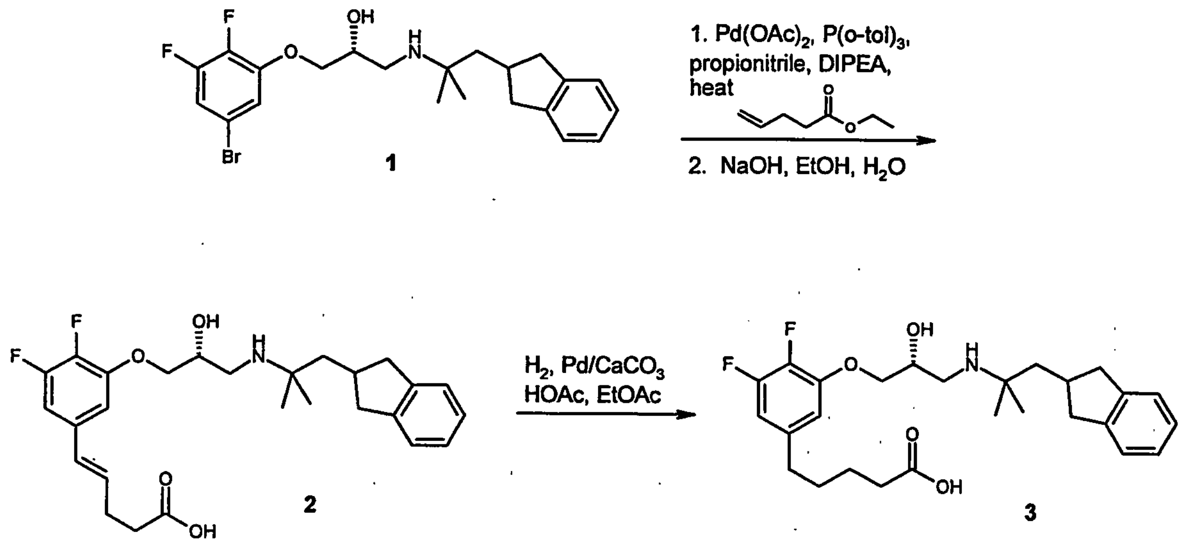


1

2

3

## Synthetic Scheme 2:

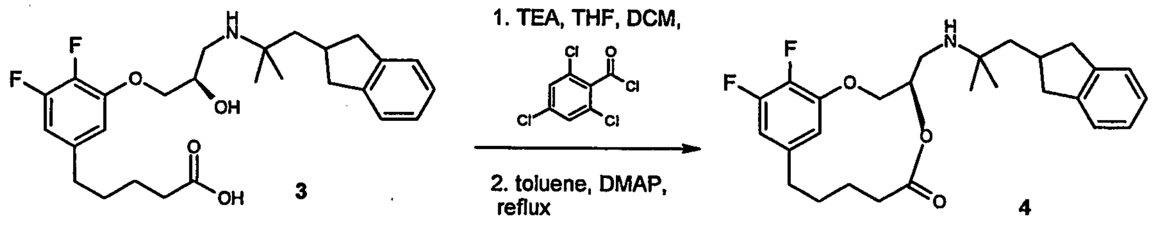


### General Preparation

Synthetic Scheme 1 outlines the preparation of pentenoic acid **3**. Heck coupling of known bromide 1 with an olefin such as ethyl-4-pentenoate provides the α,β-unsaturated ester which is saponified (without purification) with a base such as sodium hydroxide in ethanol and water to provide the pentenoic acid 2. The pentenoic acid is reduced under conditions which are common to the art such as hydrogen in the presence of a catalyst such as palladium on calcium carbonate to provide the saturated acid 3. As depicted in Synthetic Scheme 2, the acid, 3, is cyclized under conditions common to the art such as treatment with trichlorobenzoyl chloride (Yamaguchi's reagent) followed by heating in the presence of DMAP to promote macrolactonization.

In order to use a compound of Formula (I) or a pharmaceutically acceptable salt thereof for the treatment of humans and other mammals, it is normally formulated in accordance with standard pharmaceutical practice as a pharmaceutical composition.

The calcilytic compounds can be administered by different routes including intravenous, intraperitoneal, subcutaneous, intramuscular, oral, topical (transdermal), or transmucosal administration. For systemic administration, oral administration is preferred. For oral administration, for example, the compounds can be formulated into conventional oral dosage forms such as capsules, tablets, and liquid preparations such as syrups, elixirs, and concentrated drops.

Alternatively, injection (parenteral administration) may be used, e.g., intramuscular, intravenous, intraperitoneal, and subcutaneous. For injection, the compounds of the invention are formulated in liquid solutions, preferably, in physiologically compatible buffers or solutions, such as saline solution, Hank's solution, or Ringer's solution. In addition, the compounds may be formulated in solid form and redissolved or suspended immediately prior to use. Lyophilized forms can also be produced.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, bile salts and fusidic acid derivatives. In addition, detergents may be used to facilitate permeation. Transmucosal administration, for example, may be through nasal sprays, rectal suppositories, or vaginal suppositories.

For topical administration, the compounds of the invention can be formulated into ointments, salves, gels, or creams, as is generally known in the art.

The amounts of various calcilytic compounds to be administered can be determined by standard procedures taking into account factors such as the compound $IC_{50}$, $EC_{50}$, the biological half-life of the compound, the age, size and weight of the patient, and the disease or disorder associated with the patient. The importance of these and other factors to be considered are known to those of ordinary skill in the art.

Amounts administered also depend on the routes of administration and the degree of oral bioavailability. For example, for compounds with low oral bioavailability, relatively higher doses will have to be administered.

Preferably the composition is in unit dosage form. For oral application, for example, a tablet, or capsule may be administered, for nasal application, a metered aerosol dose may be administered, for transdermal application, a topical formulation or patch may be administered and for transmucosal delivery, a buccal patch may be administered. In each case, dosing is such that the patient may administer a single dose.

Each dosage unit for oral administration contains suitably from 0.01 to 500 mg/Kg, and preferably from 0.1 to 50 mg/Kg, of a compound of Formula (I) or a pharmaceutically acceptable salt thereof, calculated as the free base. The daily dosage for parenteral, nasal, oral inhalation, transmucosal or transdermal routes contains suitably from 0.01 mg to 100 mg/Kg, of a compound of Formula (I). A topical formulation contains suitably 0.01 to 5.0% of a compound of Formula (I). The active ingredient may be administered, for example, from 1 to 6 times per day, preferably once, sufficient to exhibit the desired activity, as is readily apparent to one skilled in the art.

As used herein, "treatment" of a disease includes, but is not limited to prevention, retardation and prophylaxis of the disease.

Diseases and disorders which might be treated or prevented, based upon the affected cells, include bone and mineral-related diseases or disorders; hypoparathyroidism; those of the central nervous system such as seizures, stroke, head trauma, spinal cord injury, hypoxia-induced nerve cell damage, such as occurs in cardiac arrest or neonatal distress, epilepsy, neurodegenerative diseases such as Alzheimer's disease, Huntington's disease and Parkinson's disease, dementia, muscle tension, depression, anxiety, panic disorder, obsessive-compulsive disorder, post-traumatic stress disorder, schizophrenia, neuroleptic malignant syndrome, and Tourette's syndrome; diseases involving excess water reabsorption by the kidney, such as syndrome of inappropriate ADH secretion (SIADH), cirrhosis, congestive heart failure, and nephrosis; hypertension; preventing and/or decreasing renal toxicity from cationic antibiotics (e.g., aminoglycoside antibiotics); gut motility disorders such as diarrhea and spastic colon; GI ulcer diseases; GI diseases with excessive calcium absorption such as sarcoidosis; autoimmune diseases and organ transplant rejection; squamous cell carcinoma; and pancreatitis.

In a preferred embodiment of the present invention, the present compounds are used to increase serum parathyroid hormone ("PTH") levels. Increasing serum PTH levels can be helpful in treating diseases such as hypoparathyroidism, osteosarcoma, periodontal disease, fracture, osteoarthritis, rheumatoid arthritis, Paget's disease, humoral hypercalcemia malignancy and osteoporosis.

In a preferred embodiment of the present invention, the present compounds are co-administered with an anti-resorptive agent. Such agents include, but are not limited estrogen, 1, 25 $(OH)_2$ vitamin D3, calcitonin, selective estrogen receptor modulators, vitronectin receptor antagonists, V-H+-ATPase inhibitors, src SH2 antagonists, bisphosphonates and cathepsin K inhibitors.

Another aspect of the present invention describes a method of treating a patient comprising administering to the patient an amount of a present compound sufficient to increase the serum PTH level. Preferably, the method is carried out by administering an amount of the compound effective to cause an increase in duration and/or quantity of serum PTH level sufficient to have a therapeutic effect.

In various embodiments, the compound administered to a patient causes an increase in serum PTH having a duration of up to one hour, about one to about twenty-four hours, about one to about twelve hours, about one to about six hours, about one to about five hours, about one to about four hours, about two to about five hours, about two to about four hours, or about three to about six hours.

In an alternative embodiment of the present invention, the compound administered to a patient causes an increase in serum PTH having a duration of more than about twenty four hours provided that it is co-administered with an anti resorptive agent.

In additional different embodiments, the compound administered to a patient causes an increase in serum PTH of up to two fold, two to five fold, five to ten fold, and at least 10 fold, greater than peak serum PTH in the patient. The peak serum level is measured with respect to a patient not undergoing treatment.

Composition of Formula (I) and their pharmaceutically acceptable salts, which are active when given orally, can be formulated as syrups, tablets, capsules and lozenges. A syrup formulation will generally consist of a suspension or solution of the compound or salt in a liquid carrier for example, ethanol, peanut oil, olive oil, glycerine or water with a flavoring or coloring agent. Where the composition is in the form of a tablet, any pharmaceutical carrier routinely used for preparing solid formulations may be used. Examples of such carriers include magnesium stearate, terra alba, talc, gelatin, acacia, stearic acid, starch, lactose and sucrose. Where the composition is in the form of a capsule, any routine encapsulation is suitable, for example using the aforementioned carriers in a hard gelatin capsule shell. Where the composition is in the form of a soft gelatin shell capsule any pharmaceutical carrier routinely used for preparing dispersions or suspensions may be considered, for example aqueous gums, celluloses, silicates or oils, and are incorporated in a soft gelatin capsule shell.

Typical parenteral compositions consist of a solution or suspension of a compound or salt in a sterile aqueous or non-aqueous carrier optionally containing parenterally acceptable oil, for example polyethylene glycol, polyvinylpyrrolidone, lecithin, arachis oil or sesame oil.

Typical compositions for inhalation are in the form of a solution, suspension or emulsion that may be administered as a dry powder or in the form of an aerosol using a conventional propellant such as dichlorodifluoromethane or trichlorofluoromethane.

A typical suppository formulation comprises a compound of Formula (I) or a pharmaceutically acceptable salt thereof which is active when administered in this way, with a binding and/or lubricating agent, for example polymeric glycols, gelatins, cocoa-butter or other low melting vegetable waxes or fats or their synthetic analogs.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

No unacceptable toxological effects are expected when compounds of the present invention are administered in accordance with the present invention.

The biological activity of the compounds of Formula (I) are demonstrated by the following tests:

## (I) Calcium Receptor Inhibitor Assay

Calcilytic activity was measured by determining the $IC_{50}$ of the test compound for blocking increases of intracellular $Ca^{2+}$ elicited by extracellular $Ca^{2+}$ in HEK 293 4.0-7 cells stably expressing the human calcium receptor. HEK 293 4.0-7 cells were constructed as described by Rogers et al., J. Bone Miner. Res. 10 Suppl. 1:S483, 1995. Intracellular $Ca^{2+}$ increases were elicited by increasing extracellular $Ca^{2+}$ from 1 to 1.75 mM. Intracellular $Ca^{2+}$ was measured using fluo-3, a fluorescent calcium indicator.

The procedure was as follows:

1. Cells were maintained in T-150 flasks in selection media (DMEM supplemented with 10% fetal bovine serum and 200 ug/mL hygromycin B), under 5% $CO_2$:95% air at 37 °C and were grown up to 90% confluency.
2. The medium was decanted and the cell monolayer was washed twice with phosphate-buffered saline (PBS) kept at 37 °C. After the second wash, 6 mL of 0.02% EDTA in PBS was added and incubated for 4 minutes at 37 °C. Following the incubation, cells were dispersed by gentle agitation.
3. Cells from 2 or 3 flasks were pooled and pelleted (100 x g). The cellular pellet was resuspended in 10-15 mL of SPF-PCB+ and pelleted again by centrifugation. This washing was done twice.
Sulfate- and phosphate-free parathyroid cell buffer (SPF-PCB) contains 20 mM Na-Hepes, pH 7.4, 126 mM NaCl, 5 mM KCl, and 1 mM $MgCl_2$. SPF-PCB was made up and stored at 4 °C. On the day of use, SPF-PCB was supplemented with 1 mg/mL of D-glucose and 1 mM $CaCl_2$ and then split into two fractions. To one fraction, bovine serum albumin (BSA; fraction V, ICN) was added at 5 mg/mL (SPF-PCB+). This buffer was used for washing, loading and maintaining the cells. The BSA-free fraction was used for diluting the cells in the cuvette for measurements of fluorescence.
4. The pellet was resuspended in 10 mL of SPF-PCB+ containing 2.2 uM fluo-3 (Molecular Probes) and incubated at room temperature for 35 minutes.
5. Following the incubation period, the cells were pelleted by centrifugation. The resulting pellet was washed with SPF-PCB+. After this washing, cells were resuspended in SPF-PCB+ at a density of 1-2 x 106 cells/mL.
6. For recording fluorescent signals, 300 uL of cell suspension were diluted in 1.2 mL of SPF buffer containing 1 mM $CaCl_2$ and 1 mg/mL of D-glucose. Measurements of fluorescence were performed at 37 °C with constant stirring using a spectrofluorimeter. Excitation and emission wavelengths were measured at 485 and 535 nm, respectively. To calibrate fluorescence signals, digitonin (5 mg/mL in ethanol) was added to obtain Fmax, and the apparent Fmin was determined by adding Tris-EGTA (2.5 M Tris-Base, 0.3 M EGTA). The concentration of intracellular calcium was calculated using the following equation:

$$\text{Intracellular calcium} = (F\text{-}F_{min}/F_{max}) \times K_d;\ \text{where } K_d = 400\ \text{nM}.$$

7. To determine the potential calcilytic activity of test compounds, cells were incubated with test compound (or vehicle as a control) for 90 seconds before increasing the concentration of extracellular $Ca^{2+}$ from 1 to 2mM. Calcilytic compounds were detected by their ability to block, in a concentration-dependent manner, increases in the concentration of intracellular $Ca^{2+}$ elicited by extracellular $Ca^{2+}$.

In general, those compounds having lower $IC_{50}$ values in the Calcium Receptor Inhibitor Assay are more preferred compounds. Compounds having an $IC_{50}$ greater than 50 uM were considered to be inactive. Preferred compounds are those having an $IC_{50}$ of 10uM or lower, more preferred compounds have an $IC_{50}$ of 1uM, and most preferred compounds have an $IC_{50}$ of 0.1uM or lower.

## (II) Calcium Receptor Binding Assay

HEK 293 4.0-7 cells stably transfected with the Human Parathyroid Calcium Receptor ("HuPCaR") were scaled up in T180 tissue culture flasks. Plasma membrane is obtained by polytron homogenization or glass douncing in buffer (50mM Tris-HCl pH 7.4, 1mM EDTA, 3mM $MgCl_2$) in the presence of a protease inhibitor cocktail containing 1uM Leupeptin, 0.04 uM Pepstatin, and 1 mM PMSF. Aliquoted membrane was snap frozen and stored at -80 °C. $^3H$ labeled compound was radiolabeled to a radiospecific activity of 44Ci/mmole and was aliquoted and stored in liquid nitrogen for

radiochemical stability.

A typical reaction mixture contains 2 nM $^3H$ compound ((R,R)-N-4'-Methoxy-t-3-3'-methyl-1'-ethylphenyl-1-(1-naphthyl)ethylamine), or $^3H$ compound (R)-N-[2-Hydroxy-3-(3-chloro-2-cyanophenoxy)propyl]-1,1-dimethyl-2-(4-methoxyphenyl)ethylamine 4-10 ug membrane in homogenization buffer containing 0.1% gelatin and 10% EtOH in a reaction volume of 0.5 mL. Incubation is performed in 12 x 75 polyethylene tubes in an ice water bath. To each tube 25 uL of test sample in 100% EtOH is added, followed by 400 uL of cold incubation buffer, and 25 uL of 40 nM $^3H$-compound in 100% EtOH for a final concentration of 2nM. The binding reaction is initiated by the addition of 50 uL of 80-200 ug/mL HEK 293 4.0-7 membrane diluted in incubation buffer, and allowed to incubate at 4°C for 30 min. Wash buffer is 50 mM Tris-HCl containing 0.1% PEI. Nonspecific binding is determined by the addition of 100-fold excess of unlabeled homologous ligand, and is generally 20% of total binding. The binding reaction is terminated by rapid filtration onto 1% PEI pretreated GF/C filters using a Brandel Harvestor. Filters are placed in scintillation fluid and radioactivity assessed by liquid scintillation counting.

## Example 1

**(R)-4-[(2-Indan-2-yl-1,1-dimethyl-ethylamino)-methyl]-6-oxo-2,5-dioxa-bicyclo[9.3.1]pentadeca-1(14),11(15),12-triene-14-carbonitrile**

To a stirred solution of 0.5 g (1.08 mmol) of hydroxy acid, hydrochloride salt (WO 0153254) in 21 mL of dry dichloromethane was added 0.17 mL (1.08 mmol) of 2,4,6-trichlorobenzoyl chloride and 0.38 mL of (2.37 mmol) triethylamine sequentially. The mixture was stirred for 1 h and slowly added over a 7 h period to a refluxing solution of 4-dimethylaminopyridine (0.79 g, 6.48 mmol) in 430 mL of anhydrous toluene using a syringe pump. Upon completion, the reaction was cooled to ambient temperature, diluted with ethyl acetate (1000 mL) and washed with 5% HCl (100 mL), saturated $CuSO_4$ (3 x 250 mL) and brine (200 mL) sequentially. The organic layer was dried ($Na_2SO_4$), concentrated and purified by flash column chromatography using 20% THF in dichloromethane treated with triethylamine (1%). The resulting compound was further purified by HPLC (YMC 50x20 mm, 5micron, C18 column; 40-95% $CH_3CN/H_2O$ containing 0.1% TFA, 10 minute gradient; Product $t_R$: 4.88 min.) to give 11% (0.061 g) of the product as a TFA salt.
The TFA salt of lactone (0.061 g, 0.11 mmol) was suspended in dry acetonitrile (10 mL) and treated with 2.0M HCl (0.27 mL, 5 equiv.) in ether. The reaction mixture was stirred for 15 min and concentrated. This procedure was repeated two additional times to obtain the hydrochloride salt of lactone in (0.052 g) quantitative yield.
$^1H$ NMR (500 MHz, $CDCl_3$): δ 10.3 (m, 1H); 8.25 (m, 1H); 7.43 (d, J = 7.85 Hz, 1H); 7.10-7.18 (m, 5H); 6.83 (d, J = 7.83 Hz, 1H); 5.38 (d, J = 14.03 Hz, 1H); 5.33 (d, J = 8.08 Hz, 1H); 4.36 (d, J = 14.5 Hz, 1H); 3.32 (m, 2H); 3.15 (dd, *J* = 14.9, 7.3 Hz, 1H); 2.95 (dd, *J* = 14.6, 7.4 Hz, 1H); 2.5-2.7 (m, 7H); 1.99 (d, m, 3H); 1.62 (m, 3H); 1.53 (s, 3H); 1.46 (s, 3H), 1.25 (m, 2H).
MS(m/z): 447 $(M+H)^+$.

## Example 2a

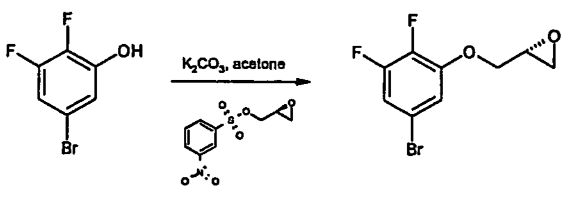


**(*R*)-2-(5-Bromo-2,3-difluoro-phenoxymethyl)-oxirane**

To an acetone solution (0.1 M, 240 mL) of commercially available 5-bromo-2,3-difluorophenol (5.0 g, 23.93 mmol) was added $K_2CO_3$ (9.92 g, 71.77 mmol), and the mixture was heated to reflux for 30 min. After cooling this mixture to RT, (2*R*)-(-)-glycidyl 3-nitrobenzenesulfonate (6.20 g, 23.93 mmol) was added, and the resulting mixture was heated to reflux overnight. After cooling to RT, the solids were removed by filtration and washed well with ethyl acetate. The filtrate was concentrated and partitioned between ethyl acetate and 1N HCl. The organic portion was washed successively with 5% $NaHCO_3$ and brine, dried ($MgSO_4$), filtered and concentrated to a solid. Purification by FCC (15% ethyl acetate/hexanes) gave the product as a white solid in 97% yield (6.19 g).

**Example 2b**

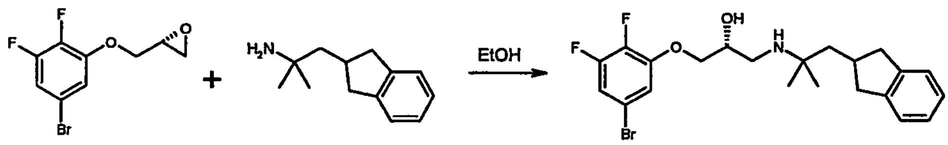


**(*R*)-1-(5-Bromo-2,3-difluoro-phenoxy)-3-(2-indan-2-yl-1,1-dimethyl-ethylamino)-propan-2-ol**

An ethanolic solution (0.2 M, 93 mL) of the above-mentioned oxirane (5 g, 18.67 mmol) and 2-indan-2-yl-1,1-dimethyl-ethylamine (free base; 3.57 g, 18.67 mmol) was heated to refux for 12 h. After solvent removal, the crude reaction mixture was purified by FCC (5% $CH_3OH/CH_2Cl_2$) to give pure product as a yellow oil (solidifies on standing) in 84% yield (7.1 g).
$^1$H NMR (400 MHz, DMSO-$d_6$): δ 9.05 (t, *J* = 9.0 Hz, 1H); 8.65 (t, *J* = 9.0 Hz, 1H); 7.40 (ddd, *J* = 9.75, 6.4, 2.2 Hz, 1H); 7.34 (ddd, *J* = 6.7, 2.0, 2.0 Hz, 1H); 7.18 (m, 2H); 7.10 (m, 2H); 6.0 (d, *J* = 4.8 Hz, 1H); 4.25 (m, 1H); 4.20 (m, 2H); 3.17 (m, 1H); 3.08 (m, 2H); 2.95 (m, 1H); 2.58 (m, 3H); 1.97 (d, *J* = 5.43 Hz, 2H); 1.39 (s, 6H).
LCMS (m/z) M+H = 454/456.

**Example 2c**

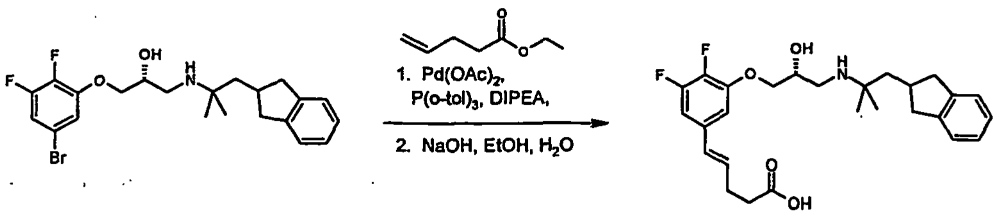


**(*E*)-5-(3,4-Difluoro-5-[(*R*)-2-hydroxy-3-(2-indan-2-yl-1,1-dimethyl-ethylamino)-propoxy]-phenyl}-pent-4-enoic acid**

The title compound was prepared in two steps in the following manner:

To a solution of the above-mentioned bromide (50.0 g, 110.13 mmol) in degassed propionitrile (0.25 M, 440 mL) were added $Pd(OAc)_2$ (1.24 g, 5.51 mmol), P(o-tol)$_3$ (5.03 g, 16.52 mmol), DIPEA (42.2 mL, 242.29 mmol), and ethyl-4-pentenoate (20.4 mL, 143.17 mmol). The reaction flask was fitted with a condenser, kept under Ar cover, and placed in a pre-heated bath (115 °C) for 3 h. After cooling to RT, the reaction mixture was filtered through Celite, and the filtrate was concentrated, partitioned between ethyl acetate and 1N HCl. The layers were separated and the organic portion was washed succesively with 1N HCl and brine, dried ($MgSO_4$), filtered and concentrated to a brown oil.

The crude residue (est. 110 mmol) was brought up in ethanol and water (0.2 M, 440 mL, 110 mL) and treated with 2N NaOH (138 mL). The reaction mixture stirred at RT for 15 h. The ethanol was removed and the aqueous portion (pH 14) was diluted up to 500 mL and extracted 4 times with 100 mL portions of diethyl ether. Aqueous HCl was added while stirring to adjust the pH to 5, causing the product come out of solution as a gum. $CH_2Cl_2$ was added, and the biphasic mixture was stirred well for 2 days resulting in the formation of a white solid suspension. The solid was isolated as the pure zwitterion by filtration (37.5 g, 72% for 2 steps).

To an acetonitrile suspension of the zwitterion product was added 2M HCl in diethyl ether. The material briefly went into solution, and then precipitated as a white crystalline solid to give the title compound as the HCl salt.
$^1$H NMR (400 MHz, DMSO-$d_6$): δ 8.9 (br m, 1H); 8.55 (br m, 1H); 7.18 (m, 2H); 7.07 (m, 5H); 6.38 (s, 1H); 5.99 (br s, 1H); 4.25 (m, 1H); 4.15 (m, 2H); 3.36 (m, 2H); 3.19 (m, 1H); 3.08 (dd, *J* = 13.3, 7.05 Hz, 2H); 2.95 (m, 1H); 2.55 (m, 3H); 2.40 (s, 2H); 1.95 (d, *J* = 5.3 Hz, 2H); 1.39 (s, 6H).
LCMS (m/z) M+H = 474.6.

**Example 2d**

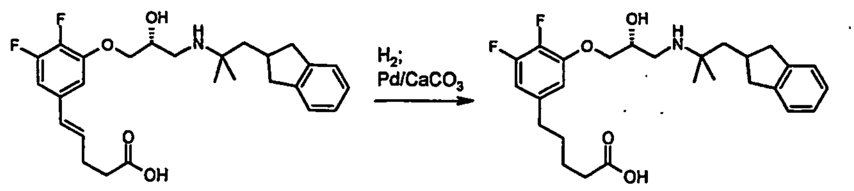


**5-(3,4-Difluoro-5-[(*R*)-2-hydroxy-3-(2-indan-2-yl-1,1-dimethyl-ethylamino)-propoxy]-phenyl}-pentanoic acid**

To a solution of the above-mentioned pentenoic acid (19.8 g, 41.82 mmol) in acetic acid (300 mL) and ethyl acetate (150 mL) was added 5% $Pd/CaCO_3$(4.0 g). The reaction flask was purged with $H_2$ and sealed under a $H_2$ balloon for 15 h. The mixture was filtered through Celite, and the filtrate was concentrated to a volume of 50 mL. Toluene (100 mL) and 2M HCl in diethyl ether (10 mL) were added, and the solution was concentrated to a foam. This procedure was repeated 3 times. The material was suspended in acetonitrile and treated with 2M HCl in diethyl ether. This solution was concentrated to dryness providing the pure HCl salt as a foam.
$^1$H NMR (400 MHz, DMSO-$d_6$): δ 8.75 (m, 1H); 8.50 (m, 1H); 7.19 (m, 2H); 7.10 (m, 2H); 6.90 (m, 2H); 5.95 (d, *J* = 4.3 Hz, 1H); 4.22 (m, 1H); 4.15 (m, 2H); 3.4 (m, 2H); 3.20 (m, 1H); 3.10 (dd, *J* = 13.8, 7.2 Hz, 2H); 2.98 (m, 1H); 2.60 (m, 3H); 2.24 (t, *J* = 7.2 Hz, 2H); 1.95 (d, *J* = 5.7 Hz, 2H); 1.56 (m, 2H); 1.51 (m, 2H),1.38 (s, 6H).
LCMS (m/z) M+H = 476.

**Example 2e**

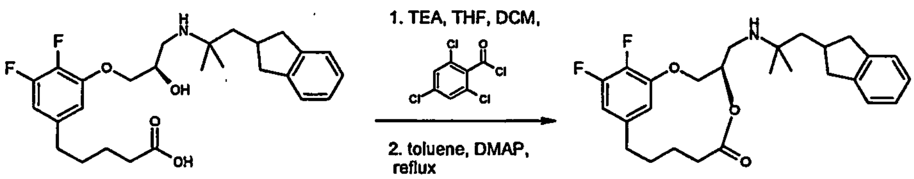


**(R)-13,14-Difluoro-4-[(2-indan-2-yl-1,1-dimethyl-ethylamino)-methyl]-2,5-dioxa-bicyclo[9.3.1]pentadeca-1(14), 11(15),12-trien-6-one**

To a stirred solution of 0.11 g (0.217 mmol) of hydroxy acid (hydrochloride salt) in 2.2 mL of dry dichloromethane was added 0.034 mL (0.217 mmol) of 2,4,6-trichlorobenzoyl chloride and 0.033 mL (0.239 mmol) of triethylamine sequentially. The mixture was stirred for 1 h at room temperature. The solution was then diluted with dry dichloromethane to a total volume of 7.0 mL and was slowly added over a 7 h period to a refluxing solution of 4-dimethylaminopyridine (1.856 g, 15.19 mmol) in 110 mL of anhydrous toluene using a syringe pump. Upon completion, the reaction was cooled to ambient temperature and stirred overnight. The resulting solution was diluted with ethyl acetate (200 mL) and washed with 5% HCl (100 mL), saturated $CuSO_4$ (3 x 100 mL) and brine (100 mL) sequentially. The organic layer was dried ($Na_2SO_4$), concentrated and purified by HPLC (YMC 75x30 mm, 5micron, C18 column; 40-95% $CH_3CN/H_2O$ containing 0.1% TFA, 10 minute gradient; Product $t_R$: 5.0 min.) to give 15% (0.015 g) of the product as a TFA salt.
The TFA salt of the lactone was suspended in dry acetonitrile and treated with 2.0M HCl in ether. The reaction mixture was stirred for 15 min and concentrated. This procedure was repeated two additional times to obtain the hydrochloride salt of the lactone.
$^1$H NMR (400 MHz, DMSO-$d_6$): δ 9.33 (m, 1H); 8.32 (m, 1H); 7.09-7.18 (m, 4H); 6.99 (d, *J* = 6.8 Hz, 1H); 6.88 (m, 1H); 5.18 (d, *J* = 8.8 Hz, 1H); 5.11 (d, *J* = 14.0 Hz, 1H); 4.42 (d, *J* = 14.0 Hz, 1H); 2.97-3.10 (m, 2H); 2.77 (m, 1H); 2.59 (m, 3H); 2.19 (m, 1H); 1.93 (d, m, 3H); 1.73 (m, 3H); 1.52-1.58 (m, 2H); 1.39 (s, 3H); 1.36 (s, 3H).
MS(m/z): 458 $(M+H)^+$.

The above description fully discloses the invention including preferred embodiments thereof. Modifications and improvements of the embodiments specifically disclosed herein are within the scope of the following claims. Without

further elaboration, it is believed that one skilled in the area can, using the preceding description, utilize the present invention to its fullest extent. Therefore the Examples herein are to be construed as merely illustrative. The embodiments of the invention in which an exclusive property or privilege is claimed are defined as follows.

## Claims

1. A compound according to formula (I) hereinbelow:

or a pharmaceutically acceptable salt thereof.

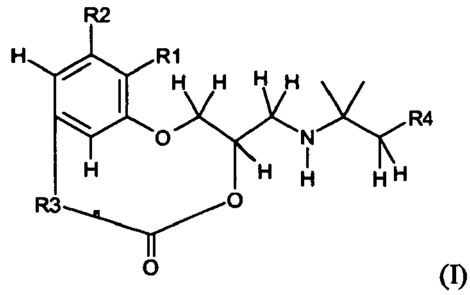

(I)

wherein:

R1 is CN, or halogen
R2 is halogen or H
R3 is $C_{3-7}$ alkyl, or $C_{3-7}$ alkenyl; optionally substituted
R4 is selected from the group consisting of aryl, fused aryl, dihydro, tetrahydro fused aryl, and heteroaryl, unsubstituted or substituted, with any substituent selected from the group consisting of OH, halogen, $C_{1-4}$ alkyl, $C_{1-4}$ alkoxy, $CF_3$, $OCF_3$, CN and $NO_2$.

2. A compound according to claim 1 selected from the group consisting of:

(R)-4-[(2-Indan-2-yl-1,1-dimethyl-ethylamino)-methyl]-6-oxo-2,5-dioxa-bicyclo[9.3.1]pentadeca-1(14),11(15),12-triene-14-carbonitrile;
(R)-13,14-Difluoro-4-[(2-indan-2-yl-1,1-dimethyl-ethylamino)-methyl]-2,5-dioxa-bicyclo[9.3.1]pentadeca-1(14),11(15),12-trien-6-one;
(R)-4- {[2-(5-Chloro-thiophen-2-yl)-1,1-dimethyl-ethylamino]-methyl}-13,14-difluoro-2,5-dioxa-bicyclo[9.3.1]pentadeca-1(14),11(15),12-trien-6-one;
(R)-4-{[2-(5-Chloro-thiophen-2-yl)-1,1-dimethyl-ethylamino]-methyl}-6-oxo-2,5-dioxa-bicyclo[9.3.1]pentadeca-1(14),11(15),12-triene-14-carbonitrile;
(R)-14-Bromo-4-[(2-indan-2-yl-1,1-dimethyl-ethylamino)-methyl]-2,5-dioxa-bicyclo[9.3.1]pentadeca-1(14),11(15),12-trien-6-one; and
(R)-14-Bromo-4-{[2-(5-chloro-thiophen-2-yl)-1,1-dimethyl-ethylamino]-methyl}-2,5-dioxa-bicyclo[9.3.1]pentadeca-1(14),11(15),12-trien-6-one.

3. A compound as claimed in claim 1 or claim 2 for use as a medicament.

4. A compound as claimed in claim 3 for use as a medicament for antagonizing a calcium receptor.

5. A compound as claimed in claim 3 for use as a medicament for the treatment of a disease or disorder **characterized by** an abnormal bone or mineral homeostasis.

6. A compound as claimed in claim 5 wherein the bone or mineral disease or disorder is selected from the group consisting of osteosarcoma, periodontal disease, fracture healing, osteoarthritis, joint replacement, rheumatoid arthritis, Paget's disease, humoral hypercalcemia, malignancy and osteoporosis.

**7.** A compound as claimed in claim 6 wherein the bone or mineral disease or disorder is osteoporosis.

**8.** A compound as claimed in claim 3 for use as a medicament for increasing serum parathyroid levels.

**9.** A compound as claimed in claim 7 or claim 8 wherein the compound is co-administered with an anti-resorptive agent.

**10.** A compound as claimed in claim 9 wherein the anti-resorptive agent is selected from the group consisting of estrogen, 1, 25 $(OH)_2$ vitamin D3, calcitonin, selective estrogen receptor modulators, vitronectin receptor antagonists, V-H+-ATPase inhibitors, src SH2 antagonists, bisphosphonates and cathepsin K inhibitors.

**11.** Use of a compound as claimed in claim 1 or claim 2 for the manufacture of a medicament for antagonizing a calcium receptor.

**12.** Use of a compound as claimed in claim 1 or claim 2 for the manufacture of a medicament for treating a disease or disorder **characterized by** an abnormal bone or mineral homeostasis.

**13.** A use as claimed in claim 12 wherein the bone or mineral disease or disorder is selected from the group consisting of osteosarcoma, periodontal disease, fracture healing, osteoarthritis, joint replacement, rheumatoid arthritis, Paget's disease, humoral hypercalcemia, malignancy and osteoporosis.

**14.** A use as claimed in claim 13 wherein the bone or mineral disease or disorder is osteoporosis.

**15.** Use of a compound as claimed in claim 1 or claim 2 for the manufacture of a medicament for increasing serum parathyroid levels.

**16.** A use as claimed in claim 14 or claim 15 wherein the compound is co-administered with an anti-resorptive agent.

**17.** A use as claimed in claim 16 wherein the anti-resorptive agent is selected from the group consisting of estrogen, 1, 25 $(OH)_2$ vitamin D3, calcitonin, selective estrogen receptor modulators, vitronectin receptor antagonists, V-H+-ATPase inhibitors, src SH2 antagonists, bisphosphonates and cathepsin K inhibitors.

**Patentansprüche**

**1.** Verbindung gemäß unten stehender Formel (I) oder ein pharmazeutisch verträgliches Salz davon:

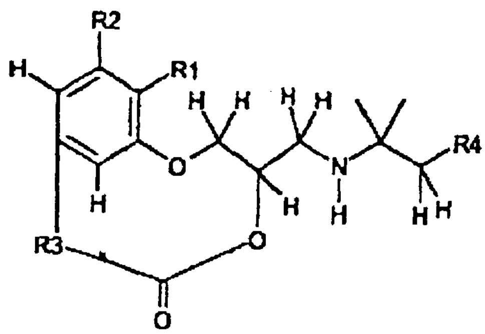

(I)

wobei:

R 1 CN oder Halogen ist,
R2 Halogen oder H ist,
R3 $C_{3-7}$Alkyl oder $C_{3-7}$Alkenyl ist; gegebenenfalls substituiert,
R4 ausgewählt ist aus Aryl, kondensiertem Aryl, Dihydro-, Tetrahydro-kondensiertem Aryl und Heteroaryl, unsubstituiert oder substituiert mit einem Substituenten ausgewählt aus OH, Halogen, $C_{1-4}$Alkyl, $C_{1-4}$Alkoxy, $CF_3$, $OCF_3$, CN und $NO_2$.

2. Verbindung gemäß Anspruch 1, ausgewählt aus:

(R)-4-[(2-Indan-2-yl-1,1-dimethyl-ethylamino)-methyl]-6-oxo-2,5-dioxa-bicyclo[9.3.1]pentadeca-1(14),11(15), 12-trien-14-carbonitril;
(R)-13,14-Difluor-4-[(2-Indan-2-yl-1,1-dimethyl-ethylamino)-methyl]-2,5-dioxa-bicyclo[9.3.1]pentadeca-1(14), 11(15),12-trien-6-on;
(R)-4-{[2-(5-Chlor-thiophen-2-yl)-1,1-dimethyl-ethylamino]-methyl}13,14-difluor-2,5-dioxa-bicyclo[9.3.1]pentadeca-1(14),11(15),12-trien-6-on;
(R)-4-{[2-(5-Chlor-thiophen-2-yl)-1,1-dimethyl-ethylamino]-methyl}-6-oxo-2,5-dioxa-bicyclo[9.3.1]pentadeca-1 (14),11(15),12-trien-14-carbonitril;
(R)-14-Brom-4-[(2-Indan-2-yl-1,1-dimethyl-ethylamino)-methyl]-2,5-dioxa-bicyclo[9.3.1]pentadeca-1(14),11 (15),12-trien-6-on; und
(R)-14-Brom-4-{[2-(5-Chlor-thiophen-2-yl)-1,1-dimethyl-ethylamino]-methyl)}-2,5-dioxa-bicyclo[9.3.1]pentadeca-1(14),11(15),12-trien-6-on.

3. Verbindung gemäß Anspruch 1 oder 2 zur Verwendung als Medikament.

4. Verbindung gemäß Anspruch 3 zur Verwendung als Medikament zum Antagonisieren eines Calciumrezeptors.

5. Verbindung gemäß Anspruch 3 zur Verwendung als Medikament zur Behandlung einer Krankheit oder einer Störung, **gekennzeichnet durch** unnormale Knochen- oder Mineralhomöostase.

6. Verbindung gemäß Anspruch 5, wobei die Knochen- oder Mineralkrankheit oder -störung ausgewählt ist aus Osteosarkom, Zahnfleischerkrankung, Bruchheilung, Osteoarthritis, Gelenkersatz, rheumatoider Arthritis, Paget-Syndrom, Pseudohyperparathyreoidismus und Osteoporose.

7. Verbindung gemäß Anspruch 6, wobei die Knochen- oder Mineralkrankheit oder -störung Osteoporose ist.

8. Verbindung gemäß Anspruch 3 zur Verwendung als Medikament zur Erhöhung des Serum-Parathyroidspiegels.

9. Verbindung gemäß Anspruch 7 oder 8, wobei die Verbindung mit einem anti-resorptiven Mittel zusammen verabreicht wird.

10. Verbindung gemäß Anspruch 9, wobei das anti-resorptive Mittel ausgewählt ist aus Östrogen, 1,25-$(OH)_2$-Vitamin D3, Calcitonin, selektiven Östrogenrezeptormodulatoren, Vitronectin-Rezeptorantagonisten, V-H+-ATPase-Inhibitoren, src-SH2-Antagonisten, Bisphosphonaten und Cathepsin-K-Inhibitoren.

11. Verwendung einer Verbindung gemäß Anspruch 1 oder 2 zur Herstellung eines Medikaments zum Antagonisieren eines Calciumrezeptors.

12. Verwendung einer Verbindung gemäß Anspruch 1 oder 2 zur Herstellung eines Medikaments zur Behandlung einer Krankheit oder einer Störung, **gekennzeichnet durch** unnormale Knochen- oder Mineralhomöostase.

13. Verwendung gemäß Anspruch 12, wobei die Knochen- oder Mineralkrankheit oder -störung ausgewählt ist aus Osteosarkom, Zahnfleischerkrankung, Bruchheilung, Osteoarthritis, Gelenkersatz, rheumatoider Arthritis, Paget-Syndrom, Pseudohyperparathyreoidismus und Osteoporose.

14. Verwendung gemäß Anspruch 13, wobei die Knochen- oder Mineralkrankheit oder -störung Osteoporose ist.

15. Verwendung einer Verbindung gemäß Anspruch 1 oder 2 zur Herstellung eines Medikaments zur Erhöhung des Serum-Parathyroidspiegels.

16. Verwendung gemäß Anspruch 14 oder 15, wobei die Verbindung mit einem anti-resorptiven Mittel zusammen verabreicht wird.

17. Verwendung gemäß Anspruch 16, wobei das anti-resorptive Mittel ausgewählt ist aus Östrogen, 1,25-$(OH)_2$-Vitamin D3, Calcitonin, selektiven Östrogenrezeptormodulatoren, Vitronectin-Rezeptorantagonisten, V-H+-ATPase-Inhibitoren, src-SH2-Antagonisten, Bisphosphonaten und Cathepsin-K-Inhibitoren.

## Revendications

1. Composé répondant à la formule (I) ci-dessous :

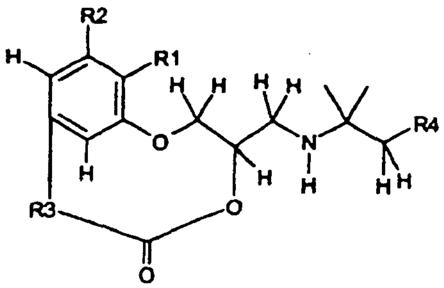

(I)

ou un de ses sels pharmaceutiquement acceptables, formule dans laquelle :

R1 représente un groupe CN ou un atome d'halogène ;
R2 représente un atome d'halogène ou de H ;
R3 représente un groupe alkyle en $C_3$ à $C_7$ ou alcényle en $C_3$ à $C_7$, facultativement substitué ;
R4 est choisi dans le groupe consistant en des groupes aryle, aryle condensé, dihydro, aryle condensé avec un groupe tétrahydro et hétéroaryle, non substitué ou substitué avec n'importe quel substituant choisi dans le groupe consistant en des substituants OH, halogéno, alkyle en $C_1$ à $C_4$, alkoxy en $C_1$ à $C_4$, $CF_3$, $OCF_3$, CN et $NO_2$.

2. Composé suivant la revendication 1, choisi dans le groupe consistant en :

le (R)-4-[(2-indane-2-yl-1,1-diméthyl-éthylamino) -méthyl]-6-oxo-2,5-dioxa-bicyclo[9.3.1]pentadéca-1(14),11(15),12-triène-14-carbonitrile ;
la (R)-13,14-difluoro-4-[(2-indane-2-yl-1,1-diméthyl-éthylamino)-méthyl]-2,5-dioxa-bicyclo[9.3.1]pentadéca-1(14),11(15),12-trién-6-one ;
la (R)-4-{[2-(5-chloro-thiophén-2-yl)-1,1-diméthyl-éthylamino]-méthyl)-13,14-difluoro-2,5-dioxa-bicyclo-[9.3.1]pentadéca-1(14),11(15),12-trién-6-one ;
le (R)-4-{[2-(5-chloro-thiophén-2-yl)-1,1-diméthyl-éthylamino]-méthyl}-6-oxo-2,5-dioxa-bicyclo[9.3.1]-pentadéca-1(14),11(15),12-triène-14-carbonitrile ;
la (R)-14-bromo-4-[(2-indane-2-yl-1,1-diméthyl-éthylamino)-méthyl]-2,5-dioxa-bicyclo[9.3.1]pentadéca-1(14),11(15),12-trién-6-one ; et
la (R)-14-bromo-4-{[2-(5-chloro-thiophène-2-yl)-1,1-diméthyl-éthylamino]-méthyl)-2,5-dioxa-bicyclo[9.3.1]pentadéca-1(14),11(15),12-trién-6-one.

3. Composé suivant la revendication 1 ou la revendication 2, destiné à être utilisé comme médicament.

4. Composé suivant la revendication 3, destiné à être utilisé comme médicament pour antagoniser un récepteur du calcium.

5. Composé suivant la revendication 3, destiné à être utilisé comme médicament pour le traitement d'une maladie ou d'un trouble **caractérisé par** une homéostase osseuse ou minérale anormale.

6. Composé suivant la revendication 5, dans lequel la maladie ou le trouble osseux ou minéral est choisi dans le groupe consistant en un ostéosarcome, une maladie périodontique, la cicatrisation d'une fracture, l'arthrose, une prothèse d'articulation, la polyarthrite rhumatoïde, la maladie de Paget, l'hypercalcémie humorale, une affection maligne et l'ostéoporose.

7. Composé suivant la revendication 6, dans lequel la maladie ou le trouble osseux ou minéral est l'ostéoporose.

8. Composé suivant la revendication 3, destiné à être utilisé comme médicament pour augmenter les taux sériques d'hormone parathyroïdienne.

**9.** Composé suivant la revendication 7 ou la revendication 8, qui est coadministré avec un agent antirésorption.

**10.** Composé suivant la revendication 9, l'agent antirésorption étant choisi dans le groupe consistant en un oestrogène, la 1,25$(OH)_2$vitamine D3, la calcitonine, des modulateurs sélectifs des récepteurs d'oestrogènes, des antagonistes du récepteur de vitronectine, des inhibiteurs de V-$H^+$-ATPase, des antagonistes de src SH2, des bisphosphonates et des inhibiteurs de cathepsine K.

**11.** Utilisation d'un composé suivant la revendication 1 ou la revendication 2, pour la production d'un médicament destiné à antagoniser un récepteur du calcium.

**12.** Utilisation d'un composé suivant la revendication 1 ou la revendication 2, pour la production d'un médicament destiné au traitement d'une maladie ou d'un trouble **caractérisé par** une homéostase osseuse ou minérale anormale.

**13.** Utilisation suivant la revendication 12, dans laquelle la maladie ou le trouble osseux ou minéral est choisi dans le groupe consistant en un ostéosarcome, une maladie périodontique, la cicatrisation d'une fracture, l'arthrose, une prothèse d'articulation, la polyarthrite rhumatoïde, la maladie de Paget, l'hypercalcémie humorale, une affection maligne et l'ostéoporose.

**14.** Utilisation suivant la revendication 13, dans laquelle la maladie ou le trouble osseux ou minéral est l'ostéoporose.

**15.** Utilisation d'un composé suivant la revendication 1 ou la revendication 2, pour la production d'un médicament destiné à augmenter les taux sériques d'hormone parathyroïdienne.

**16.** Utilisation suivant la revendication 14 ou la revendication 15, dans laquelle le composé est coadministré avec un agent antirésorption.

**17.** Utilisation suivant la revendication 16, dans laquelle l'agent antirésorption est choisi dans le groupe consistant en un oestrogène, la 1,25$(OH)_2$vitamine D3, la calcitonine, des modulateurs sélectifs des récepteurs d'oestrogènes, des antagonistes du récepteur de vitronectine, des inhibiteurs de V-$H^+$-ATPase, des antagonistes de src SH2, des bisphosphonates et des inhibiteurs de cathepsine K.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0153254 A


### Non-patent literature cited in the description


- **BROWN et al.** *Nature,* 1993, vol. 366, 574
- **NEMETH et al.** *Cell Calcium,* 1990, vol. 11, 319
- **NEMETH.** *Cell Calcium,* 1990, vol. 11, 323
- **ZAIDI.** *Bioscience Reports,* 1990, vol. 10, 493
- **ROGERS et al.** *J. Bone Miner. Res.,* 1995, vol. 10 (1), S483